# EUROPEAN PATENT SPECIFICATION

(11) **EP 2 364 694 B1**
(45) Date of publication and mention of the grant of the patent: **06.11.2013**
(21) Application number: 11157742.5
(22) Date of filing: 10.03.2011
(51) Int. Cl.: A61K 9/20, A61K 9/48, A61K 31/428

(54) **Controlled-release formulations of pramipexole**
Pramipexol-Formulierungen mit gesteuerter Freisetzung
Formulations de pramipexole à libération contrôlée

(30) Priority: 11.03.2010 TR 201001862
(43) Date of publication of application: 14.09.2011
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Turp, Hasan Ali, 34398 Istanbul (TR); Yelken, Gülay, 34398 Istanbul (TR); Öner, Levent, Ankara (TR)
(74) Representative: Sevinç, Erkan

(56) References cited:
- EP-A1- 2 135 602
- WO-A1-2004/010999
- WO-A1-2008/129043
- WO-A2-2007/054976
- WO-A2-2007/090882

## Description

### Field of Invention

The present invention relates to a method according to claim 1. It is disclosed a pharmaceutical formulation consisting of pramipexole or pharmaceutically acceptable salt or hydrate of pramipexole. Also disclosed are controlled-release formulations of pramipexole, allowing the latter to become dispersed uniformly within a matrix tablet and be used as a single daily dose.

### Background of Invention

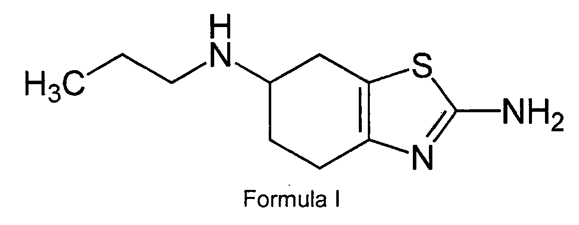

Pramipexole is a non-ergot dopamine agonist. The chemical designation of pramipexole is (S)-2-amino-4,5,6,7-tetrahydro-6-(propylamino)benzothiazole, with the chemical structure as shown in Formula I. Pramipexole has a quite high water solubility.

Immediate-release tablets of pramipexole is commercially available under the trademark Mirapex®, orally administered three times per day, and comprises 0.125 mg, 0.25 mg, 0.5 mg, 1 mg or 1.5 mg pramipexole dihydrochloride monohydrate as the active agent.

Pramipexole, along with its production processes, are disclosed in EP0186087B1 and is particularly known in treating schizophrenia and Parkinson's disease.

The application EP1531814 concerns dispersing an orally deliverable sustained-release tablet composition comprising a water-soluble salt of pramipexole in a matrix. It further comprises a hydrophilic polymer and a starch having a tensile strength of at least about 0.15 kNcm².

The application EP1536792 concerns an oral formulation of pramipexole, which, on average, and in a single dose per day *in vitro* release profile, does not dissolve more than 20% within 2 hours, and of which the *in vivo* absorption is more than 20% within 2 hours and/or more than 40% within 4 hours.

In the application EP2135602, in turn, is disclosed an extended-release tablet formulation of pramipexole comprising at least one swelling polymer other than pregelatinized starch in a matrix.

Additionally, the controlled-release formulations disclosed in the patents W02007054976, US2009004281 comprise coating substances.

Producing controlled-release tablets of pramipexole and similar active agents with high solubility rates and desired release profiles in these tablets is quite difficult. Generating release amounts at desired time intervals bears vital importance with respect to patients.

Furthermore, the amount of active agent included in pramipexole formulations is quite low and this fact makes its production difficult. It is further known that low-dose pharmaceutical compositions are problematic in providing a uniform dispersion in the formulations in which they are present. This is because it is difficult to homogenize the active agent present in the final dosage form in lower amounts and other problems may be encountered while they are compressed. This results in final dosage forms with improper content uniformity.

It is known that uniformly distributing pharmaceutical active agents with pharmaceutical auxiliaries is a desired case in formulating low-dose pharmaceutical active agents for use in patients to be treated for obtaining a proper dosage and homogeneity. Accordingly, it is further desired to provide improved processes for preparing solid oral dosage forms, which have high uniformity and disperse satisfactorily while being administered orally.

In result, there is a need towards pharmaceutical compositions, providing satisfactory distribution of pramipexole or pharmaceutically-acceptable salts, solvates, or hydrates thereof, and showing proper content uniformity and desired release profiles.

### Object and Brief Description of Invention

The present invention relates to the method according to claim 1. Disclosed are controlled-release formulations of pramipexole, eliminating all aforesaid problems and bringing additional advantages to the relevant prior art. Accordingly, the main object of the present invention is to obtain controlled-release formulations comprising pramipexole, which are stable and have a desired release profile. Another object of the present invention is to provide a uniform dispersion of pramipexole in the formulation, thereby obtaining a formulation with a proper therapeutic use range.

A further object of the present invention is to provide a uniform dispersion of pramipexole in the formulation, thereby facilitating the production process.

Yet a further aspect is to embody a controlled-release formulation of pramipexole, without requiring to apply any coating thereon.

Accordingly, a pharmaceutical formulation, which does not contain any coating substance has been developed, to achieve all objects referred to above and to emerge from the following detailed description.

Said novelty is carried out with pramipexole or a pharmaceutically acceptable salt thereof, colloidal silicone dioxide, and glyceryl behenate.

According to a preferred embodiment , pramipexole or a pharmaceutically acceptable salt of pramipexole is released by 30% at most, preferably by 20% at most in 2 hours; by 35-65% and preferably by 40-65% in 4 hours; and by 85% at least in 16 hours.

According to a preferred embodiment , glyceryl behenate is present in an amount of 5 to 90% by weight of the total composition.

According to a preferred embodiment , glyceryl behenate is present in an amount of 10 to 70% by weight of the total composition.

According to another preferred embodiment , said pramipexole is in the form of dihydrochloride monohydrate.

According to a further preferred embodiment, said formulation further comprises at least one or a mixture of xanthan gum, guar gum, a mixture of a locust bean gum-derived heterosaccharide and a dextrose-derived saccharide, or castor oil, hydrogenated castor oil, cellulose acetate phthalate, polyvinyl acetate phthalate, hydroxypropyl methyl cellulose acetate succinate, poly(methyvinyl ether/maleic acid) mono ethyl ester, poly(methyl ether/maleic acid) n-butyl ester.

In a preferred embodiment, at least one or a mixture of the following is/are used as a diluent: lactose, microcrystalline cellulose, starch, mannitol, calcium phosphate anhydrate, dibasic calcium phosphate dihydrate, calcium phosphate trihydrate, sugars, sorbitol, mannitol, xylitol, sucrose polysaccharides. Said diluent is preferably microcrystalline cellulose and dibasic calcium phosphate.

In a preferred embodiment, polyvinylprolidone is comprised as a binder.

In a preferred embodiment, magnesium stearate is comprised as a lubricant.

The present invention provides a method for preparing a pharmaceutical formulation, this method comprising the steps of
a. mixing together with colloidal silicone dioxide and pramipexole dihydrochloride monohydrate after sieving,
b. adding dibasic calcium phosphate dihydrate, glyceryl behenate, polymethacrylate, copovidone(polyvinylpyrolidone-vinyl acetate copolymer), microcrystalline cellulose into this powder mixture and mixing the resulting mixture,
c. adding magnesium stearate to the mixture after sieving and mixing the resultant mixture for a short period of time,
d. compressing the resulting mixture into tablets, or filling this powder mixture into capsules.

### Detailed Description of Invention

### Example

| **Content** | **Amount (%) (w/w)** |
|---|---|
| pramipexole dihydrochloride monohydrate | 0,05-5 |
| dibasic calcium phosphate dihydrate | 5-90 |
| glyceryl behenate and polymethacrylates | 5-90 |
| polyvinylprolidone | 0,1-30 |
| microcrystalline cellulose | 5-90 |
| colloidal silicone dioxide | 0,1-10 |
| magnesium stearate | 0,1-10 |

The formulation can be obtained via various methods. In the first method, according to the present invention, pramipexole dihydrochloride monohydrate and silicone dioxide are sieved together and mixed. Thus, pramipexole, which is quite low by volume and weight is uniformly mixed with silicone dioxide, and is surrounded by colloidal silicone dioxide particles. This allows to disperse pramipexole uniformly within the formulation. Thereafter glyceryl behenate, polymethacrylates, microcrystalline cellulose, dibasic calcium phosphate dihydrate and copovidone(polyvinylpyrolidone-vinyl acetate copolymer) is added into the first mixture and mixed together. Into this mixture formed, sieved magnesium stearate is added and the resulting mixture is mixed again. The final mixture is compressed into tablets, or filled into capsules.

In the granulation method realized via melting, not according to the present invention, pramipexole dihydrochloride monohydrate, polyvinylprolidone and dibasic calcium phosphate dihydrate are sieved together and mixed. Then glyceryl behenate is melted and this melt obtained is sprayed into the powder mixture, thereby yielding wet granules. Wet granules formed are cooled and sieved. Then, microcrystalline cellulose and polymethacrylates are mixed together with granules and then colloidal silicone dioxide is mixed with resulting powder. Into this mixture formed, sieved magnesium stearate is added and the resulting mixture is mixed again. The final mixture is compressed into tablets, or filled into capsules.

In the method realized via wet granulation, not according to the present invention, pramipexole dihydrochloride monohydrate and polyvinylpyrrolidone are dissolved in water and/or alcohol to produce a solution of pramipexole dihydrochloride monohydrate. Dibasic calcium phosphate dehydrate is added into the resulting solution, while it is mixed, mixing is continued, and it is then blended in a high-shear granulator to produce wet granules. Thereafter, the granulation step is continued with a solution of polymethacrylates. The final wet granules are sieved and dried. Then glyceryl behenate and microcrystalline cellulose are added into and and then colloidal silicone dioxide is mixed with resulting powder. Into this mixture formed, sieved magnesium stearate is added and the resulting mixture is mixed again. The final mixture is compressed into tablets, or filled into capsules.

Water, alcohol or a mixture of water-alcohol is used in the processes as a solvent.

Glyceryl behenate is characterized by not swelling upon contact with water.

A stable controlled-release formulation of pramipexole is surprisingly obtained, by which pramipexole is uniformly dispensed in the formulation and the desired release profile is achieved. A controlled-release pharmaceutical formulation is disclosed, comprising
i. pramipexole or a pharmaceutically acceptable salt of pramipexole as an active agent
ii. colloidal silicone dioxide as an excipient,
iii. glyceryl behenate as a controlled release agent and
iv. further comprising another controlled release agent which is selected from the group of xanthan gum, guar gum, a mixture of a locust bean gum-derived heterosaccharide and a dextrose-derived saccharide, or castor oil, hydrogenated castor oil, cellulose acetate phthalate, polyvinyl acetate phthalate, hydroxypropyl methyl cellulose acetate succinate, poly(methyvinyl ether/maleic acid) mono ethyl ester, poly(methyl ether/maleic acid) n-butyl ester, polymethacrylates and mixture thereof.

First controlled release agent is glyceryl behenate. Another controlled release agent is without glyceryl behenate. The weight ratio of pramipexole to glyceryl behenate is in the range of 0.01-1, this range allowing to produce a release profile desired for controlled release purposes.

It is also possible to use the following additional excipients in the formulation. Suitable binders include, but are not restricted to, at least one or a mixture of polyvinylprolidone, gelatin, sugars, glucose, natural gums, synthetic celluloses, polymethacrylate, hydroxypropyl methyl cellulose, hydroxypropyl cellulose, carboxymethyl cellulose, methyl cellulose, and other cellulose derivatives.

Suitable glidants include, but are not restricted to, at least one or a mixture of colloidal silicone dioxide, talk, aluminum silicate.

Suitable lubricants include, but are not restricted to, at least one or a mixture of sodium stearil fumarat, magnesium stearat, polyethylene glycol, stearic acid, metal stearates, boric acid, sodium chloride benzoate and acetate, sodium or magnesium lauryl sulfate.

Suitable preservatives include, but are not restricted to, at least one or a mixture of methyl paraben and propyl paraben and salts thereof (e.g. sodium or potassium salts), sodium benzoate, citric acid, benzoik acid, butylated hydroxytoluene and butylated hydroxyanisole. Suitable colorants include, but are not restricted to, at least one or a mixture of food, drug, and cosmetic (FD&C) dyes (FD&C blue, FD&C green, FD&C red, FD&C yellow, FD&C lake), ponceau, indigo Drug & cosmetic (D&C) blue, indigotine FD&C blue, carmoisine indigotine (indigo Carmine); iron oxides (e.g. iron oxide red, yellow, black), quinoline yellow, flame red, brilliant red (carmine), carmoisine, sunset yellow.

This formulation can be used to treat Parkinson's disease and restless leg syndrome.

The protection scope of the present invention is set forth in the annexed Claims and cannot be restricted to the illustrative disclosures given above, under the detailed description.

## Claims

1. A method for preparing a pharmaceutical formulation **characterized by** comprising the steps of:
a. mixing together with colloidal silicone dioxide and pramipexole dihydrochloride monohydrate after sieving,
b. adding dibasic calcium phosphate dihydrate, glyceryl behenate, polymethacrylate, copovidone(polyvinylpyrolidone-vinyl acetate copolymer), microcrystalline cellulose into this powder mixture and mixing the resulting mixture,
c. adding magnesium stearate to the mixture after sieving and mixing the resultant mixture for a short period of time,
d. compressing the resulting mixture into tablets, or filling this powder mixture into capsules.

## Patentansprüche

1. Verfahren zum Herstellen einer pharmazeutischen Formulierung, **dadurch gekennzeichnet, dass** es die Schritte umfasst:
a. Zusammenmischen von kolloidalem Siliziumdioxid und Pramipexoldihydrochloridmonohydrat nach einem Siebevorgang,
b. Zugeben von zweibasigem Kalizumphosphatdihydrat, Glycerylbehenat, Polymethacrylat, Copovidon(polyvinylpyrrolidon-Vinylacetat-Copolymer), mikrokristalliner Zellulose in diese Pulvermischung und Mischen der resultierenden Mischung,
c. Zugeben von Magnesiumstearat zu der Mischung nach einem Siebevorgang und Mischen der resultierenden Mischung für eine kurze Zeitperiode,
d. Komprimieren der resultierenden Mischung zu Tabletten oder Füllen dieser Pulvermischung in Kapseln.

## Revendications

1. Procédé de préparation d'une formulation pharmaceutique **caractérisé en ce qu'**il comprend les étapes suivantes :
a. le mélange conjointement avec du dioxyde de silicium colloïdal et du dichlorhydrate de pramipexole monohydraté après tamisage,
b. l'addition de phosphate de calcium dibasique dihydraté, de béhénate de glycéryle, de polyméthacrylate, de copovidone (copolymère de polyvinylpyrrolidone-acétate de vinyle), de cellulose microcristalline dans ce mélange pulvérulent et le mélange du mélange résultant,
c. l'addition de stéarate de magnésium au mélange après tamisage et le mélange du mélange résultant pendant une courte période de temps,
d. la compression du mélange résultant en comprimés, ou le remplissage de capsules avec ce mélange pulvérulent.
